Europäisches Patentamt

European Patent Office

Office européen des brevets

(19)

(11) Veröffentlichungsnummer: **0 054 105**
**B1**

(12) **EUROPÄISCHE PATENTSCHRIFT**

(45) Veröffentlichungstag der Patentschrift:
05.12.84

(51) Int. Cl.³: **C 08 G 63/46**

(21) Anmeldenummer: 81106925.1

(22) Anmeldetag: 04.09.81

(54) Verfahren zur Herstellung von (meth)acrylsäuremodifizierten Polyestern.

(30) Priorität: 10.12.80 AT 5988/80

(43) Veröffentlichungstag der Anmeldung:
23.06.82 Patentblatt 82/25

(45) Bekanntmachung des Hinweises auf die Patenterteilung:
05.12.84 Patentblatt 84/49

(84) Benannte Vertragsstaaten:
BE CH DE FR GB IT LI NL SE

(56) Entgegenhaltungen:
EP - A - 0 002 866
FR - A - 2 029 567

CHEMICAL ABSTRACTS, Band 83, No. 12, 22.
September 1975, Seite 44, Zusammenfassung 98556x
COLUMBUS OHIO (US)

(73) Patentinhaber: Vianova Kunstharz Aktiengesellschaft,
A-8402 Werndorf (AT)

(72) Erfinder: Leitner, Wolfgang, Dr., Fellingerstrasse 13,
A-8044 Graz (AT)
Erfinder: Tulacs, Laszlo, Dipl. Ing., Münzgrabengürtel 21,
A-8010 Graz (AT)
Erfinder: Zückert, Bertram, Dr.,
Krottendorferstrasse 90/19, A-8052 Graz (AT)

(74) Vertreter: Pitter, Robert, Dr. et al,
Postfach 191 Leechgasse 21, A-8011 Graz (AT)

ACTORUM AG

## Beschreibung

Die Erfindung betrifft ein verbessertes Verfahren zur Herstellung von (meth)acrylsäuremodifizierten Polyestern, die als Bindemittel in Formulierungen eingesetzt werden können, welche durch UV- bzw. Elektronenstrahlung gehärtet werden können.

Durch Strahlung härtbare Bindemittel auf Basis von linearen Polyestern sind z.B. aus den DE-OSS 28 38 691 und 30 00 308 bekannt. In beiden Fällen werden die Hydroxylgruppen von linearen Polyestern mit Acrylsäure verestert, um die für die Reaktion mit den als zweite Komponente eingesetzten Di- oder Polyacrylaten notwendigen Doppelbindungen einzuführen.

Die Schwierigkeit bei allen beschriebenen Verfahren liegt in der Entfernung der nicht reagierten Acrylsäure aus dem Reaktionsgemisch. Nach den genannten Literaturstellen sind dazu entweder komplizierte Waschvorgänge und/oder langdauernde Veresterungsprozesse notwendig. Gegebenenfalls können die Acrylsäurereste auch durch Vakuumdestillation entfernt werden.

Eine Schwierigkeit bei der Veresterung mit Acrylsäure besteht in der anteiligen Bildung von Polyacrylsäure in der Dampfphase bzw. im Rückflusskühlsystem, da in diesen Fällen bei Verwendung der üblichen Inhibitoren keine Stabilisierung erfolgt. Der Inhibitor befindet sich zwar in der Reaktionsmasse, nicht aber im darüberliegenden Dampfraum bzw. im Kühlsystem.

Ein weiterer Nachteil der bisher bekannten Acrylpolyester liegt in ihrer hohen Viskosität, die diese Produkte auch bei Vorliegen relativ niedrigmolekularer Grundpolyester aufweisen. In der DE-OS 28 38 691 wird zur Vermeidung dieses Nachteils vorgeschlagen, das für den Aufbau des Grundpolyesters verwendete Polyol und die Acrylsäure im Überschuss einzusetzen, so dass bei der Bildung des acrylierten Polyesters neben diesem gleichzeitig ein Polyolacrylester entsteht, welcher als reaktives Verdünnungsmittel im System vorliegt. Abgesehen von der Unkontrollierbarkeit der nebeneinander ablaufenden Prozesse, kann dadurch eine Verfahrensstufe zur Entfernung der freigebliebenen Acrylsäure, z.B. durch Auswaschen, nicht vermieden werden.

Die vorliegende Erfindung beschreibt ein Verfahren zur Herstellung von strahlenhärtbaren Bindemitteln auf der Basis von (meth)acrylsäuremodifizierten Polyestern, bei welchem die genannten technologischen und wirtschaftlichen Nachteile vermieden werden und überdies keine umweltbelastenden acrylsäurehaltigen Waschwässer anfallen.

Gegenstand der Erfindung ist demgemäss ein Verfahren zur Herstellung von (meth)acrylsäuremodifizierten Polyestern, welche durch Strahlung gehärtet werden können, welches dadurch gekennzeichnet ist, dass man einen gesättigten, vorzugsweise primäre Hydroxylgruppen und gegebenenfalls Polyätherreste aufweisenden Polyester mit einem durchschnittlichen Molekulargewicht zwischen 500 und 3000 unter Rückfluss in Gegenwart mindestens eines wasserdampfflüchtigen Polymerisationsinhibitors bei 80 bis 140°C mit 30 bis 90 Mol-% (bezogen auf die Hydroxylgruppen des Polyesters) Acrylsäure oder Gemischen aus Acrylsäure und Methacrylsäure versetzt und bis zu einem Reaktionsumsatz von mindestens 40%, vorzugsweise 50 bis 70% (bezogen auf die eingesetzte Säure) in Gegenwart eines Veresterungskatalysators reagiert, anschliessend das gebildete Reaktionswasser im Azeotropverfahren mit Hilfe eines Kohlenwasserstofflösungsmittels mit einem Siedepunkt zwischen 60 und 130°C entfernt und nach Abziehen des Lösungsmittels gegebenenfalls unter Vakuum und/oder unter Verwendung einer Destillationskolonne die freigebliebene Acrylsäure mit einer der Säuremenge äquivalenten Menge einer Mono- oder Diepoxidverbindung unter Verwendung von Triphenylphosphin als Katalysator bei 80 bis 120°C bis zu einer Säurezahl von maximal 10 mg KOH/g umsetzt.

Durch das erfindungsgemässe Verfahren werden die Nachteile der bekannten Verfahren vermieden. Überdies werden durch die kurze Kreislaufphase und die gegebenenfalls zum Einsatz gelangende Destillationskolonne die Verluste an Acrylsäure in einem äusserst niedrigen, wirtschaftlich tragbaren Rahmen gehalten.

Die Verwendung eines wasserdampfflüchtigen Inhibitors, neben welchem vorzugsweise zusätzlich auch einer der üblichen nicht wasserdampfflüchtigen Polymerisationsinhibitoren eingesetzt wird, verhindert die Polymerisation der verdampften und wiederkondensierten Acrylsäure an den Reaktorwänden und im Kühlsystem. Durch das Fehlen dieser unerwünschten Polymerisate, welche natürlich auch zum Teil in die Reaktionsmasse eingeschleppt werden, wird überdies die Viskosität der Reaktionsprodukte niedrig gehalten.

Ein weiterer Vorteil des erfindungsgemässen Verfahrens liegt in der Möglichkeit zur Verwendung von Triphenylphosphin als Katalysator bei der Umsetzung der verbliebenen Acrylsäure mit der Epoxidverbindung. Dieser Katalysator, welcher in Gegenwart von Wasser nicht wirksam ist, ist für die erfindungsgemäss hergestellten Systeme besonders vorteilhaft. Abgesehen von seiner guten katalytischen Wirkung werden durch seine Gegenwart keine Verfärbungen in den aus den Reaktionsprodukten hergestellten Überzügen hervorgerufen. Überdies zeigt er eine gute Dunkelinhibierungswirkung und wirkt trotzdem unter Einwirkung von Elektronen- oder UV-Strahlung auslösend auf die Polymerisation.

Die für das erfindungsgemässe Verfahren zur Verwendung gelangenden gesättigten Polyester weisen vorzugsweise primäre Hydroxylgruppen sowie gegebenenfalls Polyätherreste auf. Sie haben ein durchschnittliches Molekulargewicht von 500 bis 3000. Diese Vorprodukte werden in üblicher Weise durch Veresterung von Dicarbonsäuren und Diolen sowie anteilig Triolen hergestellt, wobei in erster Stufe ein linearer Polyester mit endständigen Carboxylgruppen hergestellt wird, welcher in zweiter Stufe mit einer solchen Menge eines Polyols reagiert wird, dass der Polyester eine grössere Anzahl endständiger primärer Hydroxylgruppen aufweist. Durch den später auch im Endprodukt vorliegenden Überschuss an Hydroxylgruppen wird auch eine gute Haftfestigkeit der aufgebrachten Überzüge gewährleistet.

Die Ausgangsstoffe für solche Polyester sind dem Fachmann bekannt. Besonders vorteilhaft können dabei als Dicarbonsäuren Adipinsäure und Sebacinsäure sowie o-Phthalsäure, deren Isomere und Hydrierungsprodukte eingesetzt werden. Als Diole kommen Mono-, Di- und Polyglykole vom Typ des Äthylen- oder Propinlenglykols bis zu einem Molekulargewicht von etwa 3000 bzw. Butandiol-1,4, Hexandiol-1,6 oder Neopentylglykol zum Einsatz. Als Polyole dienen Rohstoffe wie Trimethyloläthan, Trimethylolpropan, Pentaerythrit oder andere Polymethylolalkane.

Die nach bekannten Verfahren hergestellten, im wesentlichen linearen Polyester werden mit 30 bis 90 Mol-% (bezogen auf die Hydroxylgruppen des Polyesters) Acrylsäure oder Gemischen aus Acrylsäure und Methacrylsäure versetzt und in Gegenwart eines sauren Veresterungskatalysators, wie p-Toluolsulfonsäure, bis zu einem Reaktionsumsatz von mindestens 40%, vorzugsweise 50 bis 70% (bezogen auf die eingesetzte Säure) bei 80 bis 140°C verestert. Anschliessend wird dem Ansatz ein Kohlenwasserstofflösungsmittel mit einem Siedepunkt oder Siedebereich zwischen 60 und 130°C zugegeben und das bei der Veresterung entstandene Reaktionswasser möglichst quantitativ in möglichst kurzer Zeit durch azeotrope Destillation aus der Reaktionsmasse entfernt.

Bei der Veresterung der Hydroxylgruppen des Polyesters mit der ungesättigten Säure wird erfindungsgemäss ein wasserdampfflüchtiger Inhibitor zugesetzt. Solche Inhibitoren sind z.B. Nitrobenzol, Nitrosobenzol, 2-Nitro-m-Kresol, 2-Nitrodimethylanilin und andere Nitroaniline sowie Nitroanisole und Nitronaphthaline. In Mischung mit diesen wasserdampfflüchtigen Inhibitoren werden auch übliche Polymerisationsinhibitoren vom Typ des Hydrochinons oder der Hydrochinonmonoalkyläther eingesetzt. Erfindungsgemäss wesentlich ist jedoch, dass mindestens ein Teil der eingesetzten Inhibitorkombination wasserdampfflüchtig ist und damit die Polymerisation der Acrylsäure im Dampfraum bzw. im Kühlsystem auch unter technischen Bedingungen verhindert wird.

Die für die azeotrope Entfernung des Reaktionswassers geeigneten KW-Lösungsmittel sind z.B. die sogenannten Spezialbenzine, welche Siedegrenzen zwischen 60 und 140°C (bei Anilinpunkten von ca. 60°C und Kauri-Butanolwerten von ca. 3,5) aufweisen. Ebenso geeignet sind die unter den Namen Ligroin, Petroleumbenzine oder Petroläther handelsüblichen KW-Mischungen, Hexan, Heptan oder Mischungen dieser Lösungsmittel. Das eingesetzte Lösungsmittel wird anschliessend, eventuell unter Vakuum, aus dem Reaktionsgemisch entfernt, wobei Verluste an (Meth)acrylsäure gegebenenfalls durch den zusätzlichen Einbau einer Destillationskolonne verringert werden können.

Nach dem Abziehen des Lösungsmittels wird die freigebliebene Säure mit einer der festgestellten Säurezahl äquivalenten Menge einer Mono- oder Diepoxidverbindung unter Verwendung von Triphenylphosphin als Katalysator bei 80 bis 120°C bis zu einer Säurezahl von max. 10 mg KOH/g umgesetzt. Bei der Berechnung wird jeweils eine Carboxylgruppe als Äquivalent für eine Epoxidgruppe in Rechnung gestellt.

Durch die Steuerung des Veresterungsgrades der Acrylsäure einerseits und durch die Auswahl von Mono- oder Polyepoxidverbindungen lassen sich die Eigenschaften des entstehenden Harzes bestimmen. Ein hoher Veresterungsgrad bewirkt im Endprodukt niedrige Anteile von Epoxyacrylaten und in der Regel sehr gute Elastizität, während ein niedriger Veresterungsgrad einen höheren Epoxyacrylatanteil zur Folge hat. Die Filme zeigen dann hohe Oberflächenhärte, aber geringere Elastizität. Mit Hilfe des vorliegenden Verfahrens kann bei Bedarf ein sehr hoher Veresterungsgrad erreicht werden, der zu elastischen Produkten führt. Überdies kann dadurch ein hoher Anteil der meist teuren Epoxidverbindungen vermieden werden.

Als Mono- oder Polyepoxidverbindungen zur Reaktion mit der freigebliebenen Säure können z.B. Glycidylester von gesättigten oder ungesättigten Carbonsäuren, wie sie im Handel in grosser Zahl angeboten werden, eingesetzt werden. Ein günstiges Eigenschaftsbild kann dabei mit den Glycidylestern gesättigter $C_9$ bis $C_{11}$-tert. Monocarbonsäuren (z.B. Cardura E®) erhalten werden. Vorteilhaft können auch Polyepoxidverbindungen auf Basis von Glycidyläthern von Bisphenol A, Bisphenol F, deren Derivaten oder von Phenol-Novolaken eingesetzt werden.

Die so hergestellten (meth)acrylsäuremodifizierten Polyester stellen relativ niedrigviskose Harze dar. Zur Verarbeitung werden sie in bekannter Weise mit weiteren monomeren Polyacrylaten versetzt und können entweder ohne weiteres durch Elektronenstrahlen oder nach Zusatz von Photoinitiatoren durch UV-Strahlung vernetzt werden. Die Anlagen und Bedingungen für diese Härtungsmethoden sind aus der Literatur bekannt und bedürfen keiner weiteren Beschreibung.

Die mittels des erfindungsgemässen Verfahrens hergestellten Überzugsmittel ergaben Filme mit ausgezeichneten Eigenschaften, welche allen Anforderungen der Praxis voll entsprechen.

Die folgenden Beispiele dienen der weiteren Erläuterung der Erfindung. Alle Mengenangaben beziehen sich, soweit nicht ausdrücklich anders angegeben, auf Gewichtsteile bzw. Gewichtsprozente.

Herstellung der Hydroxylgruppen tragenden linearen Polyester.

Gemäss der in Tabelle 1 angegebenen Verhältnisse werden Dicarbonsäuren und Diole in einen mit Thermometer, Kreislaufeinrichtung und Inertgaszufuhr ausgestatteten Reaktor gefüllt und unter leichter Inertgaszufuhr innerhalb von 3 bis 4 Stunden auf 170°C erhitzt. Durch Zugabe von Toluol wird bei dieser Temperatur ein Kreislauf eingerichtet und die Veresterung eine weitere Stunde fortgesetzt. Nach Zugabe des Polyols wird wieder langsam die Temperatur auf 170°C gebracht und unter Kreislauf bis zu einer Säurezahl von weniger als 10 mg KOH/g verestert. Bei Verwendung flüchtiger Alkoholkomponenten hat sich der Einbau von Destillationskolonnen zur Vermeidung von Verlusten als günstig erwiesen.

Tabelle 1

|  | Polyester | | | | |
|---|---|---|---|---|---|
|  | A | B | C | D | E |
| Adipinsäure | 102 | 73 | 102 | 88 | 117 |
| THPSA [1] |  | 31 |  | 15 |  |
| Diäthylenglykol |  | 21 | 21 |  | 16 |
| Neopentylglykol | 16 | 42 |  |  | 31 |
| Polyäthylenglykol [2] |  |  |  | 60 | 90 |
| Trimethylolpropan | 121 | 54 | 107 | 121 | 121 |
| OH/1000 g | 6,7 | 6,3 | 6,0 | 5,3 | 6,1 |

(1) Tetrahydrophthalsäureanhydrid
(2) Molekulargewicht ca. 600

Beispiele 1 bis 6:

1000 Tle eines der gemäss den oben genannten Angaben hergestellten Polyester werden mit der Acrylsäure (bzw. einer Mischung aus Acryl- und Methacrylsäure) in Gegenwart der Inhibitoren und p-Toluolsulfonsäure als Veresterungskatalysator bei ca. 125°C unter Rückflusskühlung solange gehalten, bis der gewünschte Umsatz erreicht ist. Anschliessend wird das gebildete Reaktionswasser mittels des Kreislaufmittels entfernt und das Kreislaufmittel bei fallender Temperatur wieder abgezogen. Nach Bestimmung der Säurezahl wird die äquivalente Menge der Epoxidverbindung zusammen mit 1 Tl Triphenylphosphin zugegeben und die Reaktion bei ca. 120°C bis zu einer Säurezahl von weniger als 10 mg KOH/g geführt.

Die Angaben für die einzelnen Beispiele sind in Tabelle 2 zusammengefasst.

Tabelle 2

| Beispiel | 1 | 2 | 3 | 4 | 5 | 6 | 7 | 8 | 9 |
|---|---|---|---|---|---|---|---|---|---|
| Polyester 1000 g | A | A | B | B | C | C | D | E | E |
| Acrylsäure (g) | 216 | 360 | 144 | 252 | 144 | 288 | 122 | 173 | 324 |
| Methacrylsäure (g) | – | – | – | – | – | – | 69 | 52 | – |
| Mol Säure/1000 g Polyester | 3,0 | 5,0 | 2,0 | 3,5 | 2,0 | 4,0 | 2,5 | 3 | 4,5 |
| Inhibitor W (g) | 1,5 WI | 1,5 WI | 1,5 WI | 1,5 WI | 1,5 WI | 1,5 WI | 1,5 WII | 1,5 WII | 1,5 WI |
| Inhibitor NW (g) | 2,0 NWI | 2,0 NWI | 2,0 NWI | 2,5 NWII | 2,0 NWI | 2,5 NWII | 2,0 NWII | 2,0 NWI | 2,0 NWI |
| p-Toluolsulfonsäure (g) | 3,0 | 3,0 | 3,0 | 3,0 | 3,0 | 3,0 | 3,0 | 3,0 | 3,0 |
| Kreislaufmittel | KL 1 | KL 1 | KL 1 | KL 1 | KL 2 | KL 2 | KL 1 | KL 2 | KL 2 |
| Kreislauftemp. °C (max.) | 80 | 80 | 80 | 80 | 100 | 100 | 80 | 100 | 100 |
| Umsatz (Mol-% Acrylsäure) vor Kreislaufphase | 59 | 58 | 60 | 57 | 65 | 58 | 61 | 52 | 45 |
| Umsatz (Mol-% Acrylsäure) nach Kreislaufphase | 67 | 64 | 68 | 62 | 76 | 68 | 66 | 68 | 58 |
| COOH/1000 g im Reaktionsprodukt | 0,81 | 1,32 | 0,56 | 1,06 | 1,042 | 0,99 | 0,71 | 0,78 | 1,42 |
| Epoxidverbindung Menge (g) | 226 | 342 | 122 | 253 | 109 | 243 | 154 | 174 | 342 |
| Type | E 1 | E 2 | E 2 | E 2 | E 1 | E 2 | E 3 | E 3 | E 3 |
| Ausbeute (100%iges Harz) | 1440 | 1698 | 1265 | 1503 | 1252 | 1528 | 1344 | 1397 | 1663 |

Erläuterungen zur Tabelle 2:

Inhibitor W:
Wasserdampfflüchtiger Inhibitor;
WI: Nitrobenzol;
WII: 2-Nitrodimethylanilin.

Inhibitor NW:
nichtwasserdampfflüchtiger Inhibitor;
NWI: Hydrochinon;
NWII: Hydrochinonmonomethyläther.

Kreislaufmittel:
KL 1: Petroläther (60 – 80°C);
KL 2: Spezialbenzin (80 – 120°C).

Epoxidverbindung:
E 1: Glycidylester von $C_9$- bis $C_{11}$-tert. Monocarbonsäuren (4,4 Epoxidgruppen/1000 g);

E 2: Flüssiges Epoxidharz auf Bisphenol A-Basis, Epoxid-Äquivalent ca. 190 = 5,3 Epoxidgruppen/1000 g;
E 3: Epoxy-Novolak-Harz, Epoxid-Äquivalent ca. 180 = 5,5 Epoxidgruppen/1000 g.

Die Prüfung der gemäss den Beispielen hergestellten Produkte erfolgte nach Verdünnen mit Äthylhexylacrylat und Zusatz von 2,5 Gew.-% UV-Sensibilisator durch Bestrahlen einer 100 µm Schicht (Nassfilm) mit einer HTQ-7-Lampe während 12 Sekunden. In allen Fällen wurden glänzende und kratzfeste Überzüge erhalten.

**Patentansprüche**

1. Verfahren zur Herstellung von (meth)acrylsäuremodifizierten Polyestern, welche durch Strahlung gehärtet werden können, dadurch gekennzeichnet, dass man einen gesättigten, vorzugsweise primäre Hydroxylgruppen und gegebenenfalls Polyätherreste aufweisenden Polyester mit einem durchschnittlichen Molekulargewicht zwischen 500 und 3000 unter Rückfluss in Gegenwart mindestens eines wasserdampfflüchtigen Polymerisationsinhibitors bei 80 bis 140°C mit 30 bis 90 Mol-% (bezogen auf die Hydroxylgruppen des Polyesters) Acrylsäure oder Gemischen aus Acrylsäure und Methacrylsäure versetzt und bis zu einem Reaktionsumsatz von mindestens 40%, vorzugsweise 50 bis 70% (bezogen auf die eingesetzte Säure) in Gegenwart eines Veresterungskatalysators reagiert, anschliessend das gebildete Reaktionswasser im Azeotropverfahren mit Hilfe eines Kohlenwasserstofflösungsmittels mit einem Siedepunkt zwischen 60 und 130°C entfernt und nach Abziehen des Lösungsmittels, gegebenenfalls unter Vakuum und/oder unter Verwendung einer Destillationskolonne, die freigebliebene Acrylsäure mit einer der Säuremenge äquivalenten Menge einer Mono- oder Diepoxidverbindung unter Verwendung von Triphenylphosphin als Katalysator bei 80 bis 120°C bis zu einer Säurezahl von maximal 10 mg KOH/g umsetzt.

2. Verfahren nach Anspruch 1, dadurch gekennzeichnet, dass man Kombinationen aus wasserdampfflüchtigen und nicht wasserdampfflüchtigen Inhibitoren einsetzt.

**Claims**

1. Process for producing (meth)acrylic acid esters modified polyesters, curable by irradiation, characterised in that a saturated polyester, preferably carrying primary hydroxy groups and optionally polyether radicals and having an average molecular weight of between 500 and 3000, is mixed at from 80 to 140°C, in the presence of at least one polymerisation inhibitor volatile with water vapour, with from 30 to 90 mole-% (calculated on the hydroxy groups of the polyester) with acrylic acid or a mixture of acrylic acid and methacrylic acid and is reacted, in the presence of an esterification catalyst, to a ratio of at least 40%, preferably of from 50 to 70% (calculated on the acid used) subsequently the reaction water formed being distilled off, in the presence of an azeotropic esterification catalyst, with the aid of a hydrocarbon solvent with a boiling point of between 60 and 130°C, and, subsequently to stripping the solvent, optionally by stripping the solvent by vacuum, or using a distillation column, the acrylic acid having remained free being reacted with a quantity of a mono- or diepoxy compound equivalent to the free acid, at from 80 to 120°C, to an acid value of a maximum of 10 mg KOH/g, using triphenylphosphine as catalyst.

2. Process according to claim 1, characterised in that a combination of inhibitors is used which are either volatile with water vapour or non-volatile with water vapour.

**Revendications**

1. Procédé pour la fabrication de polyesters modifiés par l'acide (méth)acrylique, qui peuvent être durcis par rayonnement, caractérisé par le fait que l'on fait réagir, sous reflux à une température de 80 à 140°C, en présence d'au moins un inhibiteur de polymérisation évaporable à la vapeur d'eau, un polyester saturé, d'un poids moléculaire moyen compris entre 500 et 3000 et comportant de préférence des groupes hydroxyle primaires et, le cas échéant, des restes polyéther avec 30 à 90% en moles (par rapport aux groupes hydroxyle du polyester) d'acide acrylique ou de mélanges d'acide acrylique et méthacrylique jusqu'à un taux de conversion d'au moins 40%, de préférence 50 à 70% (par rapport à l'acide mis en œuvre) en présence d'un catalyseur d'estérification, que l'on élimine ensuite l'eau de réaction formée par un procédé azéotropique à l'aide d'un solvant hydrocarboné ayant un point d'ébullition compris entre 60 et 130°C et que, après avoir éliminé le solvant, le cas échéant sous vide et/ou en utilisant une colonne de distillation, on fait réagir l'acide acrylique qui est resté libre avec une quantité de composé époxyde ou diépoxyde équivalant à la quantité d'acide en utilisant de la triphénylphosphine en tant que catalyseur à une température de 80 à 120°C jusqu'à l'obtention d'un indice d'acide de 10 mg KOH/g au maximum.

2. Procédé selon la revendication 1, caractérisé par le fait que l'on met en œuvre des combinaisons d'inhibiteurs évaporables à la vapeur d'eau et d'inhibiteurs non évaporables à la vapeur d'eau.